# EUROPEAN PATENT APPLICATION

(11) **EP 4 036 055 A1**
(43) Date of publication of application: **03.08.2022**
(21) Application number: 21154189.1
(22) Date of filing: 29.01.2021
(51) Int. Cl.: C01B 3/38, C12P 3/00, F23G 5/00

(54) **PROCESS FOR PRODUCING A HYDROGEN-CONTAINING PRODUCT GAS USING ENERGY FROM WASTE**

(71) Applicant: Hitachi Zosen Inova AG, 8005 Zürich (CH)
(72) Inventor: MITITELU, Mihai, 8005 Zürich (CH); HEMRLE, Jaroslav, 8005 Zürich (CH); WIEGERS, Jan, 8005 Zürich (CH)
(74) Representative: Schaad, Balass, Menzl & Partner AG

(57) **Abstract**

The invention describes a process and a system for producing a hydrogen-containing product gas 44, 45 using energy from waste. The process comprises the steps of feeding waste 12 from a waste supply 10 to a boiler 22 of a waste incinerator; combusting said waste 12 to produce heat, supplying a methane comprising gas 30 from a gas supply and steam 36 from a steam supply 34 to a reactor 32 of a steam reforming unit; providing heat produced in the boiler 22 to the reactor 32 of the steam reforming unit; and producing a product gas 44, 45 comprising hydrogen by the reaction of methane comprising gas 30 and steam 36 supported by heat provided by the combustion of waste 12 in the boiler 22.

## Description

The present invention relates to a process including steam reforming of a reaction gas containing methane for the production of hydrogen. More specifically, the invention relates to a process that allows a production of hydrogen from renewable feedstock materials and enables an overall improved efficiency of the steam methane reforming process.

To reduce the carbon footprint of an individual or company, new sources of energy and ways to use this energy are explored. Over the last two decades, the use of electricity as operating power instead of fossil fuels, such as oil or petrol, have gained increased attention. One problem with electric driven processes is that the electric power is not easy to store. It is possible to store kinetic energy in reservoir lakes that can later be used in combination with water turbines to provide electric power. However, it is much more convenient to have an energy carrier in form of a transportable substance, such as the fossil fuels coal, oil and natural gas.

In addition, an electric car is only as environmentally friendly as the source of energy that is used for the electric power production to fuel the car. In other words, E-mobility is useless if the electricity is originally produced in gas or coal power plants. In consequence, if we want to reduce the exploitation of natural resources, particularly oil, the use of electric power must go hand in hand with an energy production from renewable (non-fossil) resources.

A promising CO₂-neutral energy source is the combustion and/or fermentation of biomass, since this biomass has bound CO₂ from the environment. The biomethane resulting from fermentation and/or combustion of biomass is therefore CO₂-neutral.

Another ideal candidate for environmentally friendly energy source is hydrogen since it is CO₂-neutral if produced by a CO₂-neutral energy source and can be stored for use in a fuel cell in various occasions such as cars or households. On the downside, contrary to e.g. coal or crude oil, hydrogen is not readily available but must be produced.

For the production of hydrogen, the so-called "steam methane reforming process" is routinely used in the chemical processing industry to obtain pure hydrogen (or a synthesis gas comprising a mixture of hydrogen and carbon monoxide) from a reaction gas containing methane or another hydrocarbon source and steam. The steam methane reforming reaction - or just short: reforming reaction - is generally represented by the following equation (I)

CH₄ + 2H₂O → CO₂ + 4H₂ (I)

This reaction is strongly endothermic ΔHᵣ = 206 kJ/mol. The required reaction heat is usually applied by partial oxidation of methane according to the following equation (II)

2CH₄ + O₂ → 2CO + 4H₂ (II)

This reaction is exothermic ΔHᵣ = -71 kJ/mol.

The reforming process is generally carried out at a high temperature and pressure to facilitate the reaction between the steam and methane, and in the presence of a usually nickel-based catalyst supported on alumina or another suitable material.

Classically, the catalytic steam reforming is followed by a step of conversion to steam, namely the "Water Gas Shift (WGS) Reaction", that maximizes the production hydrogen by the following reaction according to the following equation (III)

CO + H₂O → CO₂ + H₂ (III)

This reaction is exothermic ΔHᵣ = -41 kJ/mol. The resulting hydrogen gas streams can then be used for a variety of purposes, e.g. as alternative fuel in motor vehicle engines.

Although steam reforming is an effective way of producing hydrogen, there are still quite a challenges to overcome: For once, steam reforming is a strongly endothermic process and requires a high reaction temperature (> 750°C). For the reforming reaction to take place, the required catalyst is usually located in tubes placed in a furnace, which is heated through combustion of a fuel to provide the required heat. This heat is usually either provided by the combustion of methane or by using part of the hydrogen produced in the steam reformer as fuel for the furnace. In the latter case, no external fuel is required, but it lowers the net amount of hydrogen that is available for industrial uses. In addition, large and complex reforming furnaces are generally needed. This complexity of the components and the associated costs generally require large production facilities for making the steam reforming process economically viable. There is thus a need for more compact reforming technologies that allow smaller productions to be profitable.

It is therefore an object of the present invention to provide a process for producing hydrogen with an improved overall energy efficiency and improved yield of hydrogen, such that is also economically viable for smaller production facilities. In a particularly preferred embodiment, the process uses renewable, i.e. non-fossil, organic feedstock material for the production of hydrogen.

This object is achieved by the process of the present invention as defined in Claim 1. Preferred embodiments are subject of the dependent claims.

In accordance with Claim 1, the present invention refers to a process for producing a hydrogen-containing product gas using energy from waste comprising the steps of:
a) Feeding waste from a waste supply to a boiler of a waste incinerator and combusting said waste to produce heat and supplying a methane comprising gas from a gas supply and steam from a steam supply to a reactor of a steam reforming unit;
b) Providing heat produced in the boiler in step a) to the reactor;
c) Producing a product gas comprising hydrogen by a reaction of methane comprising gas and steam provided in step a), whereby the reaction is supported by heat provided in step b).

In the context of the present application, the following definitions apply:
The term "waste" refers to organic and inorganic compounds that can be combusted in a waste incinerator.

The term "boiler" refers to a housing of the waste incinerator in which the combustion of waste takes place.

The term "methane comprising gas" refers to any gas that comprises aside from potential other components methane (CH₄). The methane comprising gas can be of renewable or non-renewable origin. Non-renewable sources of methane are e.g. natural gas from landfills or coal beds. An example of a renewable methane source is the process of anaerobic digestion by means of which organic matter is digested to produce inter alia biomethane.

The term "steam" is a synonym of water vapor and refers the gaseous phase of water.

The term "reactor" refers to a housing in which the methane comprising gas and the steam can react to the hydrogen comprising product gas.

Compared to the commonly used steam reforming plants known in the art, the process of the present invention involves the combustion of waste in a boiler of an incineration plant in order to produce heat that can then be used to drive the steam reforming process. As such, the inventive process eliminates the need for using hydrogen and/or methane as combustion fuel to activate the endothermic steam-methane reforming process. Therefore, the ratio of produced mol of hydrogen per supplied mol of methane is higher, which results in an improved energy balance of the process compared with the state of the art.

The hydrogen producing reaction of step c) is generally employed by using a steam reforming catalyst. Suitable steam reforming catalysts are well known in the art. These include transition metal catalysts, such as rhodium, nickel, cobalt, platinum, palladium, ruthenium and iridium. The catalyst may be supported or unsupported.

In a particularly preferred embodiment of the invention the methane comprising gas of step a) comprises biomethane produced in an anaerobic digestor by the digestion of organic material.

The term "anaerobic digestor" (AD) hereby refers to a housing in which microorganisms break down biodegradable material in the absence of oxygen in a sequence of processes. Such anaerobic digestors are commonly known in the art.

With the usage of biodegradable material (also known as organic material) the production of hydrogen can be labelled renewable, CO₂-neutral or green hydrogen. A preferred organic material for the anaerobic digestor is comingled food waste such as supermarket left overs, catering and kitchen waste, biowaste, garden waste such as cut gras or branches, market and vegetable waster and wood waste. Since it is produced from biodegradable material, the gas originating from an anaerobic digestor is generally called biogas. This biogas usually comprises biomethane as well as about 15% - 60% of CO₂ and other contaminants. These contaminants are preferably removed with a pressure-temperature swing absorption (PTSA) or a membrane in a process called biogas upgrading. Suitable upgrading processes are well known in the art. The upgraded biomethane preferably has a methane concentration of at least 80%, more preferably above 95%. A suitable anaerobic digestor produces between 200 and 350 kg/h of methane .

It is further preferred that prior to step a), waste from the waste supply is separated into an organic waste fraction and an inorganic waste fraction; the organic waste fraction being delivered to the anaerobic digestor for digestion and the inorganic waste fraction being delivered to the boiler for combustion.

The separation of waste prior to being fed to the boiler allows to use a broader range of waste. While a waste incineration plant can process all kind of waste as long as it is combustible, the anaerobic digester can generally only process organic waste. By first separating the waste into an organic and an inorganic waste fraction, a single waste source can be used to supply both the waste incinerator and the anaerobic digester. This allows for an improved energy balance since said waste can serve as fuel for the production of heat and as starting material for the production of biomethane, i.e. for producing two of the three main components in the steam reforming reaction.

Part of the heat produced in the boiler or part of the biomethane produced in the anaerobic digestor may also be used in other areas of the inventive system. For instance, the anaerobic digestor runs preferably between 30°C to 38°C for a mesophilic digestion or between 49 to 57°C for thermophilic digestion. To provide this temperature condition the anaerobic digestor could theoretically be heated by the combustion of some of the produced biomethane or by use of another thermal source, like electricity. However, this would lead to a decreased methane net balance of the anaerobic digestor or to increased operation costs. It is therefore more preferred that a part of the combustion heat produced in step a) - or part of excess heat produced by the reaction in the reactor in step c) - is transferred to the anaerobic digester to support the digestion. If more biomethane is produced in the anaerobic digestor than required for the steam reforming process, such excess biomethane may be stored and transported for use as an energy source in distant processes.

As mentioned, biomethane is part of the biogas produced through the digestion of organic waste in the anaerobic digestor. The material remaining after the digestion is called residual waste material. In a preferred embodiment of the invention this residual waste material is transported to the boiler for combustion. The residual waste may be in a solid state or in form of a slurry. Although solid material is easier combustible, the incineration process generally tolerates it if at least part of the waste is provided in form of a slurry, which safes the costs for equipment needed for drying the waste material prior to supplying it to the boiler. The residual waste material or so-called "leftover" from the anaerobic digestion cannot be further used for the production of biomethane, but it still possesses a thermal value that can be fully utilized by combustion of the residual waste in the boiler.

In a preferred embodiment of the invention the heat produced in the boiler in step a) is used in the steam supply to produce steam. This further improves the energy balance of the hydrogen production, since no external energy source - such as electric power or the combustion of biomethane or hydrogen - has to be used to produce steam for the steam-methane reforming process. The produced heat can either be transported to a separate steam supply by a heat transfer media, or preferably the steam supply is placed within the boiler of the waste incinerator. By placing the steam supply in the boiler, the heat can directly be transferred to the steam supply without using a heat transfer media, which reduces heat loss during the transfer and enables a more compact construction of the incinerator-steam reformer system, which will be described in more detail further below.

In a preferred embodiment of the invention the heat produced in the boiler in step a) is transferred to the steam reforming unit in step b) via a heat transfer medium, whereby said heat transfer medium is a fluid, preferably air, and circulates through pipes of heat exchangers provided in the boiler and the reactor. Transport of the heat transfer media in a closed circuit has the advantage that there is no direct contact of the heat transfer medium (generally air) with the combustion flue gases of the boiler or the steam-methane gas mixture in the reactor. This provides stable reaction conditions in the boiler and the reactor and allows that heat transfer can be precisely controlled by regulating the amount of transported heat transfer medium.

As an alternative it is also preferred that in step b), the heat produced in the boiler in step a) is transferred to the steam reforming unit via a heat transfer medium, whereby said heat transfer medium is combustion flue gas and said flue gas is fed from the boiler through or around pipes running through the reactor. Similarly, to the embodiment mentioned above a heat transfer medium is used to transfer heat from the boiler to the reactor. However, since in this embodiment flue gases are used as heat transfer medium, these flue gases are preferably transported in a closed pipe through the reactor and subsequently to a flue gas treatment unit or back to the boiler. This construction allows a more direct heat transfer to the reactor since the hot flue gas is directly used as a heat transfer medium instead of transferring the heat from the hot flue gases to another transfer medium, e.g. air, that is then transferred to the reactor.

Preferably the reactor of the steam reforming unit is placed within the boiler of the waste incinerator, such that heat from the boiler is directly transferred to the reactor via thermal conduction through walls of the reactor. In this third embodiment of heat transfer, the heat is directly transferred via the walls of the reactor, thereby further reducing the heat loss that would occur when using a heat transfer medium. Depending on the position of the reactor within the boiler the amount of heat that is provided by the boiler to the reactor can be adjusted.

Depending on the size, construction or shape of the boiler it is not always possible to place the reactor inside of the boiler. In this case, the boiler and the reactor may share one common wall, which still allows a direct heat transfer and reduces the loss of heat that would occur with the provision of a heat transfer media. Depending on the placing of the reactor and area of the shared common wall, the amount of heat that is provided by the boiler to the reactor can be adjusted.

Alternatively, the reactor may be integrated into the boiler wall(s). For instance, the reactor may comprise one or more pipes running within the boiler wall(s), or the rector itself forms one or more walls of the boiler. This has the advantage that the space within the boiler is not restricted by the reactor. In addition, it provides a large surface area for heat exchange.

Preferably the reactor is supplied with heat of a temperature of 800 - 1000°C, preferably with 850 - 900°C. It is further preferred that 5 - 10% of the heat produced in the boiler is transported to the reactor. A temperature range of 800 - 1000°C for the above-mentioned steam-methane reforming process have shown to provide high yields of produced hydrogen. The ideal temperature range is 850 - 900°C. Within this temperature range the reaction generally provided the highest yields of produced hydrogen. As mentioned it is preferred that 5 - 10% of the heat from the boiler is transferred to the reactor. With the usual size of the boilers of incineration plants, 5-10% of the heat produced in the boiler is generally sufficient for driving the reforming reaction in the reactor without having a negative impact on the efficiency of combustion. Up to an additional 5% of the total heat produced in the boiler (i.e. up to a combined total of 15% of the heat produced) can be further used for other processes, e.g. to heat the anaerobic digestor as mentioned above anaerobic digestor, without reducing the efficiency of combustion in the boiler.

Alternatively, heat may also be extracted from other sources, i.e. other than the boiler. In a preferred embodiment of the invention in a step d) heat is extracted from the product gas of step c) and supplied to the anaerobic digestor and/or used to increase the temperature of the methane comprising gas prior to being supplied to the reactor. For an efficient reaction of the methane comprising gas with steam in the reactor, the temperature of the methane comprising gas has to be increased to about 300°C - 500°C. This heating of the methane comprising gas can efficiently be achieved in a heat exchanger, in which heat of the product gas is transferred to the methane comprising gas. This is further advantageous since the product gas in the reactor has a temperature of about 800°C - 1000°C and needs to be cooled down to enable the "Water Gas Shift Reaction" according to equation (III) mentioned above. Therefore, a heat transfer from the product gas to the methane comprising gas and/or to the anaerobic digestor decreases the need for external energy for heating and cooling and thus improves the energy balance of the inventive process .

In a further aspect, the present invention also refers to a system for the production of hydrogen in accordance with the above-described process. Said system comprises
- a waste incinerator including a boiler for the combustion of waste;
- a steam reforming unit comprising a reactor for the production of hydrogen;
- a steam supply; and
- a gas supply for suppling a methane comprising gas,
wherein the reactor is connected to the boiler to allow a heat transfer from the boiler to the reactor.

The present invention thus provides an integrative system including a boiler or a waste incinerator and a reactor of a steam reforming unit. The connection between the boiler and the reactor is such that it enables transferring heat from the boiler to the reactor to support the above mentioned hydrogen production reaction in the reactor without the need to use an external energy source, such as methane or hydrogen. For the same reasons discussed above in connection with the inventive process, the construction of the inventive system leads to a significant improvement of the energy balance and saves costs for construction and operation of the system.

Preferably the boiler of the waste incinerator and the reactor of the steam reforming unit are connected via at least one heat transfer pipe for transferring heat from the waste incinerator to the reactor with the aid of a heat transfer medium running through the pipe. The heat transfer medium is preferably transported in a closed circuit, such that there is no direct contact of the heat transfer medium with the steam and methane gas mixture in the reactor. This provides stable reaction conditions in the reactor and the transported heat can be precisely controlled by adjusting the amount of transported heat transfer medium.

In a preferred embodiment of the system, the reactor of the steam reforming unit is located inside of the boiler, enabling direct heat transfer from the waste incinerator to the reactor via thermal conduction through reactor walls. In this alternative of heat transfer, the heat is directly transferred through the walls of the reactor. This reduces the loss of heat that generally occurs when a heat transfer medium is used for the heat transfer. Depending on the position of the reactor within the boiler the amount of heat that is provided by the boiler to the reactor can be adjusted.

In a further preferred embodiment of the system the reactor is integrated into the boiler wall or forms at least part of one or more of the boiler wall(s). In this case, the reactor preferably comprises or consists of at least one reactor pipe that runs through the wall(s) of the boiler. If the reactor consists of a number of pipes placed within the reactor wall(s), there is a large surface area that can be used for the heat transfer.

In a further alternative, the boiler and the reactor may share one common wall, but are placed adjacent to each other. The heat transfer therefore takes place via the common wall and still provides the benefit of less heat loss compared to the use of a heat transfer medium. The amount of heat transfer occurring between the boiler and the reactor is dependent on the thickness and size of the common wall.

Preferably the gas supply is an anaerobic digestor for the production of biomethane. This enables the production of biomethane from the organic waste, which counts as a CO₂ neutral energy source and as a renewable (non-fossil) hydrogen feedstock source, compared with the steam methane reforming from natural gas sources, which is a fossil hydrogen feedstock source.

In a preferred embodiment the system includes a separation device for the separation of waste from the waste supply into an organic waste fraction to be delivered to the anaerobic digester and an inorganic waste fraction to be delivered to the boiler.

As mentioned before, the usage of a separation device allows to use a broader range of waste. Typically, a waste incineration processes all kind of waste that is suitable for combustion. The prior separation of the waste into organic waste that can be used to produce biomethane in the anaerobic digestor and into inorganic waste that can be used for combustion in the boiler provides an improved energy balance, since one waste source can provide combustion heat as well as biomethane than can be stored and transported and used as an energy source in other processes.

The invention is now described in more detail on the basis of exemplary embodiments that are represented in the purely schematic figures, in which:
- Figure 1: shows a schematic view of a first preferred embodiment of the process for hydrogen production;
- Figure 2: shows a schematic view of a second preferred embodiment of the process for hydrogen production;
- Figure 3: shows a schematic view of a third preferred embodiment of the process for hydrogen production;
- Figure 4: shows a schematic view of a fourth preferred embodiment of the process for hydrogen production; and
- Figure 5: shows a schematic view of a preferred embodiment of the steam supply.

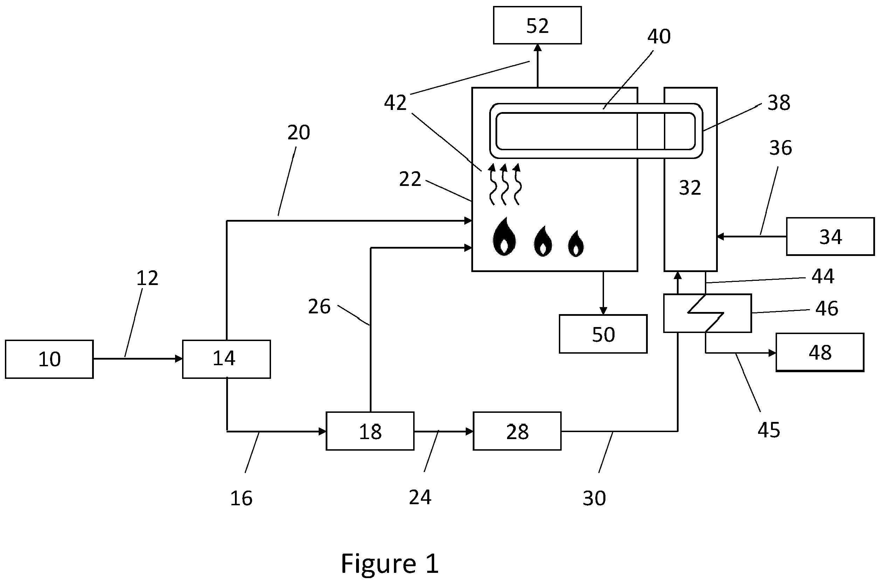

Figure 1 shows a schematic view of a preferred embodiment of the inventive process for hydrogen production. A waste supply 10 supplies unsorted waste 12 to a waste separation device 14, in which the waste is separated into organic waste 16 that can be digested in an anaerobic digestor 18, and into inorganic waste 20 that can be combusted in a boiler 22 of a waste incinerator (not shown). In the anaerobic digestor 18, the organic waste 16 is digested through anaerobic digestion, i.e. a process in which organic matter is broken down by bacteria in the absence of oxygen to produce i) raw biogas 24 comprising biomethane 30 and ii) residual waste 26. The residual waste 26 is transported to the boiler 22 for combustion, whereas the raw biogas 24 is upgraded in an upgrading device 28 to biomethane 30. The biomethane 30 is transported to a reactor 32 of a steam reforming unit (not shown). A steam supply 34 supplies steam 36 to the reactor 32. The reactor 32 and the boiler 22 are connected by at least one heat transfer medium pipe 38 that is filled with a heat transfer medium 40, here air. The combustion of the inorganic waste 20 and the residual waste 26 in the boiler 22(the combustion is indicated by flame symbols), hot flue gases 42 are produced. These hot flue gases 42 emit heat that is taken up by the heat transfer medium 40 in the pipe 38. The hot transfer medium 40 is then transported to the reactor 32, where heat from the hot transfer medium is released into the reactor to support the steam reforming reaction of steam 36 and biomethane 30 to produce a hot product gas 44 comprising hydrogen.

The hot product gas 44 and the biomethane 30 from the anaerobic digestor 18 are both delivered to a heat exchanger 46 to allow a heat exchange between the hot product gas 44 and the biomethane 30, such that the temperature of the biomethane 30 is increased while the temperature of the hot product gas 44 is decreased. The temperature reduction of the hot product gas 44 is important to allow the hydrogen content of the product gas 44 to be increased in a subsequent water-gas-shift reaction. The product gas 45 is then transported from the heat exchanger 46 to a hydrogen separation unit 48 to isolate the hydrogen from the product gas 45.

The remnants of the combusted waste from the boiler 22 are transported to a combustion residue storage 50, whereas the flue gases 42 from the boiler 22 are transported to a treatment unit 52 for cleaning, before they are released into the environment.

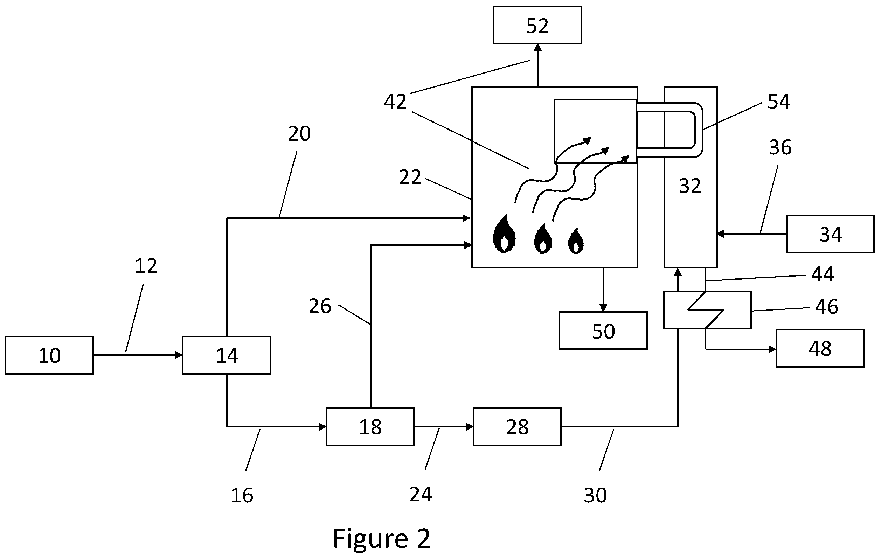

Figure 2 shows a schematic view of a second preferred embodiment of the inventive process for hydrogen production. Compared with the embodiment shown in Figure 1, the boiler 22 comprises an open heat transfer pipe 54 through which the heat transfer medium (here: flue gases 42) transfer the heat from the boiler 22 to the reactor 32 to support the steam reforming reaction of steam 36 and biomethane 30 to produce the hot product gas 44 comprising hydrogen.

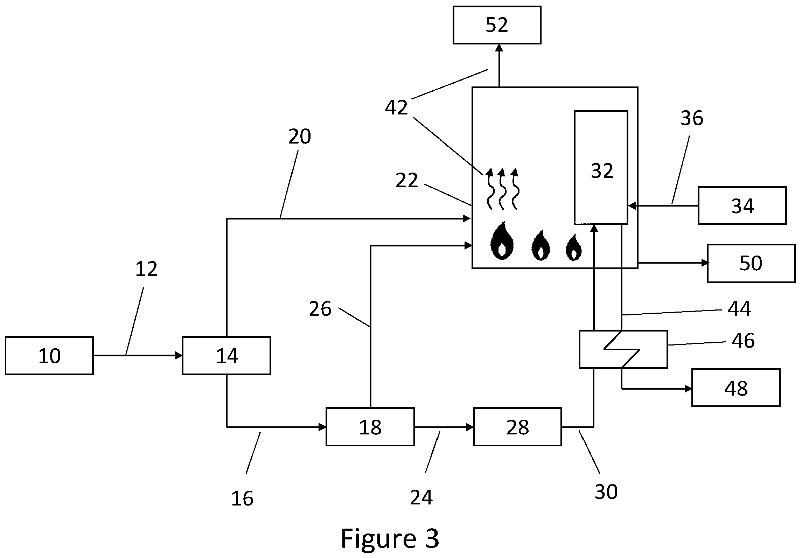

Figure 3 shows a schematic view of a third preferred embodiment of the inventive process for hydrogen production. Compared with the embodiment disclosed in Figures 1 and 2, the reactor 32 is now placed within the boiler 22. In such an embodiment, the hot flue gases 42 transfer the combustion heat directly to the walls of the reactor 32 and the heat reaches the reactor 32 via thermal conduction to support the steam reforming reaction of steam 36 and biomethane 30 to produce the hot product gas 44 comprising hydrogen.

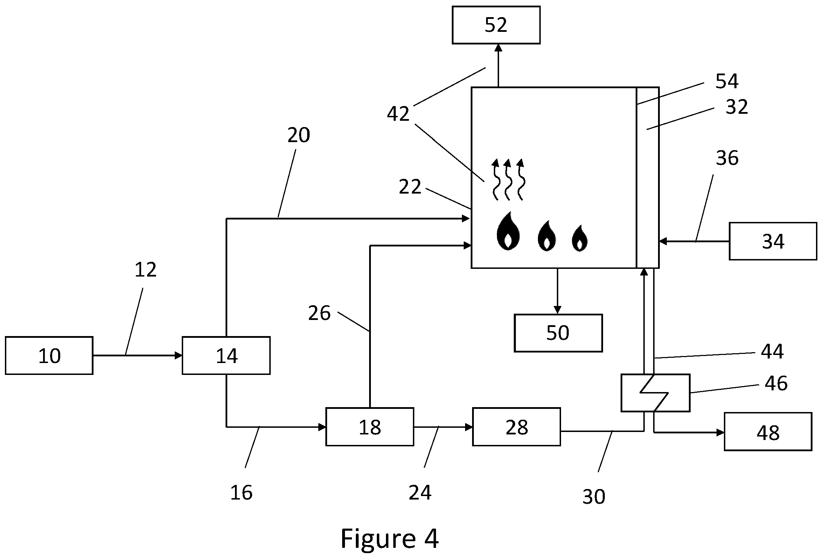

Figure 4 shows a schematic view of a fourth preferred embodiment of the inventive process for hydrogen production. Compared with the embodiment disclosed in Figures 1 to 3, the reactor 32 is here either integrated into a wall 54 of the boiler 22 or the boiler and the reactor share a common wall 54. In such an embodiment, combustion heat is given off into the reactor 32 via the hot flue gases 42 that transfer the combustion heat directly to the wall 54. Within the reactor 32 the heat supports the steam reforming reaction of steam 36 and biomethane 30 to produce the hot product gas 44 comprising hydrogen. If the reactor 32 is integrated into the wall 54 of the boiler 22, the reactor may consist of one or more pipes (not shown) that run inside, i.e. within, the wall 54.

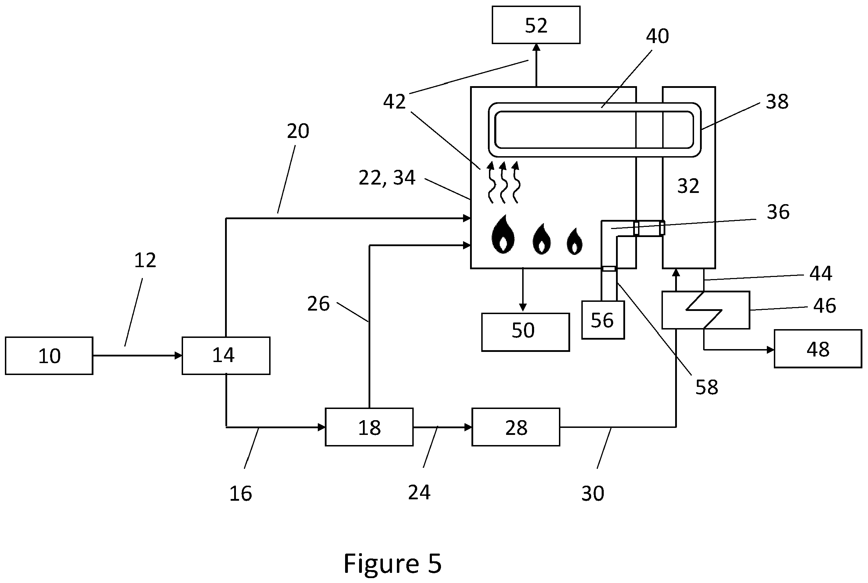

Figure 5 shows a schematic view of a preferred embodiment of the inventive process for hydrogen production, in which the boiler 22 also works as a steam supply 34. A water supply 56 supplies water into a pipe 58 running through the boiler 22 to the reactor 32. In the boiler 22 the temperature of the water is increased to the point of evaporation such that steam 36 is generated and thus provided to the reactor 32.

## Claims

1. Process for producing a hydrogen-containing product gas (44, 45) using energy from waste comprising the steps of:
a) Feeding waste (12) from a waste supply (10) to a boiler (22) of a waste incinerator and combusting said waste (12) to produce heat, and supplying a methane comprising gas (30) from a gas supply and steam (36) from a steam supply (34) to a reactor (32) of a steam reforming unit;
b) Providing heat produced in the boiler (22) in step a) to the reactor (32) of the steam reforming unit;
c) Producing a product gas (44, 45) comprising hydrogen by a reaction of methane comprising gas (30) and steam (36) provided in step a), whereby the reaction is supported by heat provided in step b) .

2. Process according to Claim 1, **characterized in that** the methane comprising gas (30) of step a) comprises biomethane (30) produced in an anaerobic digestor (18) by the digestion of organic material (16) .

3. Process according to Claim 2, **characterized in that** prior to step a), waste (12) from the waste supply (10) is separated into an organic waste fraction (16) and an inorganic waste fraction (20); the organic waste fraction (16) being delivered to the anaerobic digestor (18) for digestion and the inorganic waste fraction (20) being delivered to the boiler (22) for combustion.

4. Process according to one of the Claims 2 or 3, **characterized in that** part of the combustion heat produced in step a) or part of excess heat produced by the reaction in the reactor in step c) is transferred to the anaerobic digestor (18) to support the digestion.

5. Process according to one of Claims 2 to 4, **characterized in that** the boiler (22) of the waste incinerator is supplied with a residual waste material (26) remaining after the anaerobic digestion of the organic material (16) in the anaerobic digestor (18), for combustion.

6. Process according to one of the Claims 1 to 5, **characterized in that** the heat produced in the boiler (22) in step a) is used in the steam supply (34) to produce steam (36).

7. Process according to one of the Claims 1 to 6, **characterized in that** the steam supply (34) is placed within the boiler (22) of the waste incinerator.

8. Process according to one of the Claims 1 to 8, **characterized in that** in step b), the heat produced in the boiler (22) in step a) is transferred to the steam reforming unit via a heat transfer medium (40), whereby said heat transfer medium (40) is a fluid, preferably air and circulates through pipes of heat exchangers provided in the boiler (22) and the reactor (32).

9. Process according to one of the Claims 1 to 8, **characterized in that** in step b), the heat produced in the boiler (22) in step a) is transferred to the steam reforming unit via a heat transfer medium, whereby said heat transfer medium is combustion flue gas (42) and said flue gas (42) is fed from the boiler (22) through pipes running through or around the reactor (32).

10. Process according to one of the Claims 1 to 8, **characterized in that** the reactor (32) of the steam reforming unit is placed within or is integrated into the wall(s) (54) of the boiler (22) of the waste incinerator, such that heat from the boiler (22) is directly transferred to the reactor (32) via thermal conduction through walls of the reactor (32).

11. Process according one of the Claims 1 to 10, **characterized in that** the reactor (32) is supplied with heat of a temperature of 800 - 1000°C, preferably with 850 - 900°C.

12. Process according to one of Claims 1 to 11, **characterized in that** in a step d), heat is extracted from the product gas (44) of step c) and supplied to the anaerobic digestor (18) and/or used to increase the temperature of the methane comprising gas (30) prior to being supplied to the reactor (32).

13. System for the production of hydrogen in accordance with the process defined in one of Claims 1 to 12, said system comprising:
- a waste incinerator including a boiler (22) for the combustion of waste (12);
- a steam reforming unit comprising a reactor (32) for the production of hydrogen;
- a steam supply (34); and
- a gas supply for suppling a methane comprising gas (30);
wherein the reactor (32) is connected to the boiler (22) to allow a heat transfer from the boiler (22) to the reactor (32).

14. System according to Claim 13, **characterized in that** the boiler (22) of the waste incinerator and the reactor (32) of the steam reforming unit are connected via at least one heat transfer pipe (38) for transferring heat from the boiler (22) to the reactor (32) with the aid of a heat transfer medium (40) running through the pipe (38).

15. System according to Claim 13 or 14, **characterized in that** the reactor (32) of the steam reforming unit is located inside of the boiler (22) or the reactor (32) forms at least part of the boiler wall(s), thereby enabling direct heat transfer from the boiler (22) to the reactor (32) via thermal conduction through reactor walls.

16. System according to one of the Claims 13 to 15, **characterized in that** the gas supply is an anaerobic digestor (18) for the production of biomethane (30).

17. System according to one of the Claims 13 to 16, further **characterized by** a separation device (14) for the separation of waste (12) from the waste supply (10) into an organic waste fraction (16) to be delivered to the anaerobic digestor (18) and an inorganic waste fraction (20) to be delivered to the boiler (22).
